# EUROPÄISCHE PATENTANMELDUNG

(11) **EP 1 025 822 A1**
(43) Veröffentlichungstag der Anmeldung: **09.08.2000**
(21) Anmeldenummer: 99101688.2
(22) Anmeldetag: 08.02.1999
(51) Int. Cl.: A61F 13/15

(54) **Saugkörper für Hygieneartikel**

(71) Anmelder: Paul Hartmann Aktiengesellschaft, 89522 Heidenheim (DE)
(72) Erfinder: Malowaniec, Krysztof D., 89522 Heidenheim (DE)
(74) Vertreter: Dreiss, Fuhlendorf, Steimle & Becker, Patentanwälte

(57) **Zusammenfassung**

Beschrieben wird ein Saugkörper für einen absorbierenden Hygieneartikel für den einmaligen Gebrauch zur Aufnahme und Speicherung von Körperflüssigkeiten, insbesondere Urin, mit einer im Gebrauch körpernah angeordneten Aufnahme- und Verteilerschicht (2) mit intravernetzten Zellstofffasern und mit einer im Gebrauch körperfern angeordneten Speicherschicht (4) mit natürlichen, nicht vernetzten Zellstofffasern und superabsorbierenden Materialien, wobei die flächenhafte Ausdehnung der körpernahen Aufnahme- und Verteilerschicht kleiner ist als die der Speicherschicht und in allen Richtungen innerhalb der Umrandung der Speicherschicht angeordnet ist. Dabei ist vorgesehen, dass die Aufnahme- und Verteilerschicht (2) aus intravernetzten Zellstofffasern mit einem ersten Retentionswert zwischen 0,6 und 0,9 g_{Fl}/g_{Faser} und 8-15 Gew.-% superabsorbierenden Polymermaterialien besteht und die Speicherschicht (4) aus nicht vernetzten Zellstofffasern mit einem zweiten Retentionswert zwischen 1,0 und 1,4 g_{Fl}/g_{Faser} und wenigstens 20 Gew.-% superabsorbierenden Polymermaterialien besteht.

## Beschreibung

Die Erfindung betrifft einen Saugkörper für einen absorbierenden Hygieneartikel für den einmaligen Gebrauch zur Aufnahme und Speicherung von Körperflüssigkeiten, insbesondere Urin, mit einer im Gebrauch körpernah angeordneten Aufnahmeund Verteilerschicht mit intravernetzten Zellstofffasern und mit einer im Gebrauch körperfern angeordneten Speicherschicht mit natürlichen, nicht vernetzten Zellstofffasern und superabsorbierenden Materialien, wobei die flächenhafte Ausdehnung der körpernahen Aufnahme- und Verteilerschicht kleiner ist als die der Speicherschicht und in allen Richtungen innerhalb der Umrandung der Speicherschicht angeordnet ist.

Ein derartiger Saugkörperaufbau ist bspw. aus der WO-A-91/11163 bekannt. Diese Druckschrift lehrt, die körpernahe Aufnahme- und Verteilerschicht so auszubilden, dass sie zu 50 bis 100 Gew.-% intravernetzte Zellulosefasern und zu 0 bis 50 Gew.-% ein Bindemittel für diese Fasern umfasst. Die Aufnahme- und Verteilerschicht soll vorzugsweise frei von superabsorbierenden Materialien sein. Die Ausführungsbeispiele offenbaren eine Aufnahme- und Verteilerschicht aus 92 Gew.-% intravernetzten Zellulosefasern und 8 Gew.-% nicht vernetzten Zellulosefasern; ferner ein Ausführungsbeispiel einer Aufnahme- und Verteilerschicht, die zu 100 % aus intravernetzten Zellulosefasern besteht, sowie ein Ausführungsbeispiel mit einer Aufnahme- und Verteilerschicht, die 55 Gew.-% intravernetzte Zellulosefasern und 45 Gew.-% thermoplastische Polyethylen Microfasern umfasst, wobei letztere das Bindemittel bilden. Bei sämtlichen Ausführungsbeispielen sind entsprechend der Lehre der Druckschrift in der Aufnahme- und Verteilerschicht keine superabsorbierenden Materialien enthalten.

Aus der GB-A-2 215 609 ist ein nicht gattungsgemäßer Saugkörperaufbau bekannt, wonach die obere Aufnahme- und Verteilerschicht sich im wesentlichen über die gesamte flächenhafte Erstreckung eines Hygieneartikels ausdehnt und eine gegenüber dieser sehr viel kleinere Schicht vollständig überdeckt, die nach der Lehre dieser Druckschrift überwiegend im vorderen Bereich des Hygieneartikels vorgesehen und dort eine lokale hohe Absorptionskapazität zur Verfügung stellt. Der Faseranteil beider Schichten besteht aus intravernetzten Zellulosefasern.

Die Druckschrift lehrt mithin eine sehr spezielle Anordnung durch lokale Anreicherung von superabsorbierenden Materialien.

Der vorliegenden Erfindung liegt die Aufgabe zugrunde, bei einem Saugkörper der eingangs genannten Art das Rücknässungsverhalten zu verbessern. Bei Druckbelastung auf einen Saugkörper eines Hygieneartikels bspw. beim Sitzen, kann noch in der Aufnahme- und Verteilerschicht enthaltene Flüssigkeit, die nicht in den darunter befindlichen Saugkörper geleitet wurde an die Körperoberfläche des Benutzers gelangen und dort ein als unangenehm empfundenes Nässegefühl auslösen. Der Saugkörper soll desweiteren auf kostengünstige Weise und möglichst einfach aufgebaut sein.

Diese Aufgabe wird bei einem Saugkörper der eingangs genannten Art erfindungsgemäß dadurch gelöst, dass die Aufnahme- und Verteilerschicht aus intravernetzten Zellstofffasern mit einem zweiten Retentionswert zwischen 0,6 und 0,9 g_{Fl}/g_{Faser} und 8-15 Gew.-% superabsorbierenden Polymermaterialien besteht und die Speicherschicht aus nicht vernetzten Zellstofffasern mit einem ersten Retentionswert zwischen 1,0 und 1,4 g_{Fl}/g_{Faser} und wenigstens 20 Gew.-% superabsorbierenden Polymermaterialien besteht.

Der zweischichtige Saugkörper ist besonders einfach und damit leicht herstellbar ausgebildet, indem der Faseranteil der Aufnahme- und Verteilerschicht aus intravernetzten natürlichen Zellstofffasern und derjenige der Speicherschicht aus nicht vernetzten natürlichen Zellstofffasern besteht. Als einzige weitere Komponente ist ein superabsorbierendes Polymermaterial enthalten. Der Retentionswert der Fasern wird nach einem nachfolgend zu beschreibenden Verfahren bestimmt. Es wurde festgestellt, dass durch den Einsatz von superabsorbierenden Materialien in einem gewichtsprozentualen Anteil von 8 bis 15, vorzugsweise von 8 bis 12, insbesondere 10 bis 12 Gew.-% in der Aufnahme- und Verteilerschicht und bei einer vorstehend erwähnten Wahl des Faseranteils ein sehr gutes Aufnahme- und Verteilerverhalten der körperzugewandten Schicht als auch hervorragende Rücknässungseigenschaften erreicht werden. Durch den Faseranteil in Form von intravernetzten natürlichen Zellulosefasern ist ein vorteilhaftes Porenvolumen der körperzugewandten Aufnahme- und Verteilerschicht auch im nassen Zustand erreicht. Intravernetzte natürliche Zellulosefasern haben die Eigenschaft bei Flüssigkeitszufuhr aufzubauschen, so dass ihre Dichte bei Einnässung verringert wird, was bedeutet, dass die einzelnen Fasern ein größeres Volumen einnehmen. Durch diese Vorgänge wird das Porenvolumen und damit die Flüssigkeitsaufnahmerate vergrößert.

Es wurde nun erfindungsgemäß festgestellt, dass durch einen verhältnismäßig hohen Anteil von superabsorbierenden Materialien in der Aufnahme- und Verteilerschicht dieser Vorgang durch das Quellverhalten der superabsorbierenden Materialien noch unterstützt wird, die die Fasern stützend voneinander abheben und aufbauschen. Das Porenvolumen wird noch stärker vergrößert, was wiederum zu einer noch höheren Aufnahmekapazität während des Flüssigkeitsanfalls führt. Die Verbindung eines verhältnismäßig hohen SAP-Gehalts in Verbindung mit vernetzten natürlichen Zellstofffasern mit einem Retentionswert zwischen 0,6 und 0,9 g_{Fl}/g_{Faser} führt dazu, dass das befürchtete Gelblocking, welches in Verbindung mit höheren SAP-Gehalten im Stand der Technik vermutet wurde, gerade nicht auftritt. Wenn aber die Aufnahme- und Verteilerschicht nur einen geringen oder gar keinen Anteil an superabsorbierenden Materialien umfasst, so ist der Restwassergehalt dieser Schicht und damit das Rücknässungsverhalten nicht zufriedenstellend. Durch den erfindungsgemäßen Saugkörperaufbau wird das Rücknässungsverhalten erheblich verbessert. In der Aufnahme- und Verteilerschicht verbliebene Flüssigkeit wird durch den hohen Gehalt an superabsorbierenden Materialien wirksam und vollständig gebunden, und bei Ausübung von Druck auf den Saugkörper wird keine Flüssigkeit an die Haut des Benutzers gedrückt.

Das Flüssigkeitsrückhaltevermögen der vernetzten und nicht vernetzten natürlichen Zellstofffasern wird durch den folgenden Zentrifugentest durch Angabe des Retentionswerts bestimmt. Eine Schicht aus zu untersuchenden Zellstofffasern wird im trockenen Zustand gewogen, um deren Masse in Gramm zu ermitteln. Die Prüflinge werden dann 30 Minuten lang vollständig in einer einprozentigen Natriumchlorid-Lösung von demineralisiertem Wasser als Prüflösung eingetaucht und anschließend 4 Minuten bei 276-facher Erdbeschleunigung geschleudert. Danach werden die Prüflinge wiederum gewogen, um die Masse einschließlich der gebundenen Flüssigkeit zu bestimmen. Die Masse der aufgenommenen oder gebundenen Flüssigkeit ergibt sich daher aus der Differenz der nach dem Schleudern bestimmten Masse und der Trockenmasse des zu untersuchenden Fasermaterials. Dividiert man diese Differenz durch die Trockenmasse, so erhält man den Retentionswert in g_{Fl}/g_{Faser}.

Gegenstand der Erfindung ist auch ein absorbierender Hygieneartikel für den einmaligen Gebrauch mit einem flüssigkeitsdichten Rückblatt, einem flüssigkeitsdurchlässigen Deckblatt und einem dazwischen angeordneten Saugkörper, der dadurch gekennzeichnet ist, dass der Saugkörper nach der vorliegenden Erfindung ausgebildet ist und die Speicherschicht des Saugkörpers sich gleichmäßig über Vorder-, Rück- und Schrittbereich des Hygieneartikels erstreckt, so dass sie etwa symmetrisch zu einer mittigen Querlinie des Schrittbereichs angeordnet ist.

Weitere Merkmale, Einzelheiten und Vorteile der Erfindung ergeben sich aus den beigefügten Patentansprüchen, und aus der zeichnerischen Darstellung und nachfolgenden Beschreibung einer bevorzugten Ausführungsform eines erfindungsgemäßen Saugkörpers. In der Zeichnung zeigt:
- Figur 1: eine schematische Schnittansicht eines erfindungsgemäßen Saugkörpers;
- Figur 2: eine schematische perspektivische Ansicht eines Hygieneartikels.

Die Figur zeigt einen erfindungsgemäßen Saugkörper, bestehend aus einer, bei Verwendung in einem Hygieneartikelkörper zugewandten Aufnahme- und Verteilerschicht 2 und einer Speicherschicht 4. Die Aufnahme- und Verteilerschicht 2 besteht aus intravernetzten natürlichen Zellstofffasern mit 8 bis 12 Gew.-% eines superabsorbierenden Polymermaterials. Die Speicherschicht 4 ist aus nichtvernetzten natürlichen Zellstofffasern, vorzugsweise desselben Typs mit einem Retentionswert von zwischen 1,0 und 1,4 g_{Fl}/g_{Faser} und wenigstens 20 Gew.-% eines superabsorbierenden Polymermaterials gebildet. Die Speicherschicht 4 umfasst eine der Aufnahme- und Verteilerschicht 2 zugewandte SAP-haltige Teilschicht 6 und eine im Gebrauch körperabgewandte SAP-freie Teilschicht 8.

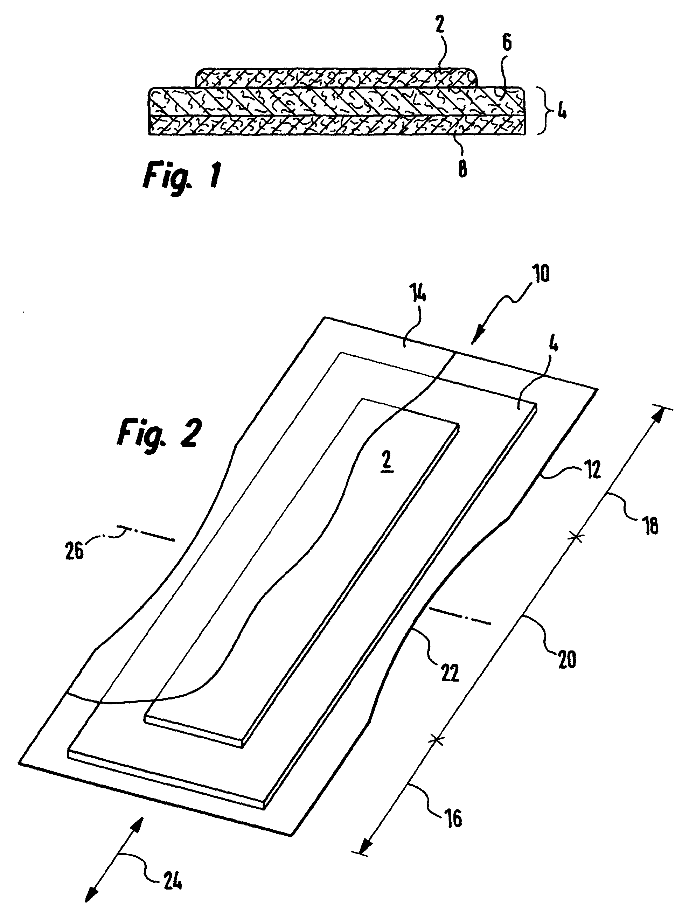

Figur 2 zeigt einen insgesamt mit dem Bezugszeichen 10 bezeichneten Hygieneartikel in Form einer Windel mit einem flüssigkeitsdichten Rückblatt 12, einem angedeuteten flüssigkeitsdurchlässigen Deckblatt 14 und einem dazwischen angeordneten Saugkörper, bestehend aus körperfern angeordneter Speicherschicht 4 und körpernah zu liegen kommender Aufnahme- und Verteilerschicht 2, die wie vorstehend in Verbindung mit Figur 1 beschrieben ausgebildet sind. Man erkennt, dass die Speicherschicht 4 sich gleichmäßig über einen Vorder- 16, Rück- 18 und Schrittbereich 20 des Hygieneartikels 10 erstreckt, wobei der Schrittbereich 20 als derjenige Bereich bezeichnet und angesehen wird, über den sich Beinausschnitte 22 in Längsrichtung 24 des Hygieneartikels erstrecken. Demgemäß ist der bis die Speicherschicht 4 symmetrisch zu einer mittig verlaufenden Querlinie 26 als Symmetrieachse ausgebildet.

## Patentansprüche

1. Saugkörper für einen absorbierenden Hygieneartikel für den einmaligen Gebrauch zur Aufnahme und Speicherung von Körperflüssigkeiten, insbesondere Urin, mit einer im Gebrauch körpernah angeordneten Aufnahme- und Verteilerschicht (2) mit intravernetzten Zellstofffasern und mit einer im Gebrauch körperfern angeordneten Speicherschicht (4) mit natürlichen, nicht vernetzten Zellstofffasern und superabsorbierenden Materialien, wobei die flächenhafte Ausdehnung der körpernahen Aufnahme- und Verteilerschicht kleiner ist als die der Speicherschicht und in allen Richtungen innerhalb der Umrandung der Speicherschicht angeordnet ist, **dadurch gekennzeichnet**, dass die Aufnahme- und Verteilerschicht (2) aus intravernetzten Zellstofffasern mit einem ersten Retentionswert zwischen 0,6 und 0,9 g_{Fl}/g_{Faser} und 8-15 Gew.-% superabsorbierenden Polymermaterialien besteht und die Speicherschicht (4) aus nicht vernetzten Zellstofffasern mit einem zweiten Retentionswert zwischen 1,0 und 1,4 g_{Fl}/g_{Faser} und wenigstens 20 Gew.-% superabsorbierenden Polymermaterialien besteht.

2. Saugkörper nach Anspruch 1, dadurch gekennzeichnet, dass die körpernahe Aufnahme- und Verteilerschicht (2) zu 8 - 12 Gew.-% aus superabsorbierenden Polymermaterialien besteht.

3. Saugkörper nach Anspruch 2, dadurch gekennzeichnet, dass die körpernahe Aufnahme- und Verteilerschicht (2) zu 10 - 12 Gew.-% aus superabsorbierenden Polymermaterialien besteht.

4. Saugkörper nach Anspruch 1, 2 oder 3, dadurch gekennzeichnet, dass die Speicherschicht (4) eine Teilschicht (8) auf der von der Aufnahme- und Verteilierschicht (2) abgewandten Seite aufweist, die frei von superabsorbierenden Materialien ist.

5. Absorbierender Hygieneartikel für den einmaligen Gebrauch mit Vorderbereich, Rückbereich und dazwischen liegendem Schrittbereich, mit einem flüssigkeitsdichten Rückblatt, einem flüssigkeitsdurchlässigen Deckblatt und einem dazwischen angeordneten Saugkörper, dadurch gekennzeichnet, dass der Saugkörper nach einem oder mehreren der vorstehenden Ansprüche ausgebildet ist und die Speicherschicht (2) des Saugkörpers sich gleichmäßig über Vorder-, Rück- und Schrittbereich des Hygieneartikels erstreckt.
